# EUROPEAN PATENT SPECIFICATION

(11) **EP 2 794 904 B1**
(45) Date of publication and mention of the grant of the patent: **06.09.2017**
(21) Application number: 12858832.4
(22) Date of filing: 21.12.2012
(51) Int. Cl.: C12P 19/34, C12Q 1/68, C12Q 1/70, C12N 15/10

(54) **AMPLIFICATION OF A SEQUENCE FROM A RIBONUCLEIC ACID**
AMPLIFIZIERUNG EINER SEQUENZ AUS EINER RIBONUCLEINSÄURE
AMPLIFICATION D'UNE SÉQUENCE PROVENANT D'UN ACIDE RIBONUCLÉIQUE

(30) Priority: 22.12.2011 US 201161579512 P
(43) Date of publication of application: 29.10.2014
(73) Proprietor: Ibis Biosciences, Inc., Carlsbad, CA 92008 (US)
(72) Inventor: MOTLEY, Stanley, Carlsbad California 92010 (US); ESHOO, Mark W., San Diego California 92130 (US)
(74) Representative: Chapman, Desmond Mark
(86) International application number: PCT/US2012/071303
(87) International publication number: WO 2013/096798

(56) References cited:
- WO-A1-99/29907
- US-A- 5 545 522
- US-A- 5 554 516
- US-A- 6 132 997
- US-A1- 2010 151 471
- BOTERO ET AL.: 'Poly(A) polymerase modification and reverse transcriptase PCR amplification of environmental RNA.' APPL. ENVIRON. MICROBIOL. vol. 71, no. 3, March 2005, pages 1267 - 1275, XP055157403

## Description

### FIELD

The present invention relates to methods for tagging, amplifying, purifying, and/or characterizing of ribonucleic acid (RNA) in a sample. In particular, methods are provided for preparing viral RNA of unknown sequence from a sample for subsequent analysis.

### BACKGROUND

Next Generation sequencing technologies are revolutionizing the process for identifying pathogens in a sample. However, these technologies are typically limited in that they require a relatively large amount of input DNA to sequence, whereas important clinical/forensic/environmental samples of interest may contain low levels of important viral pathogen nucleic acid. Further compounding this issue is the fact that many important pathogenic viruses have genomes that are composed of RNA.

US 5,545,522 describes processes wherein complementary deoxyribonucleic acid (cDNA) is synthesized from a ribonucleic acid (RNA) sequence using a complementary primer linked to an RNA polymerase promoter region complement and then anti-sense RNA (aRNA) is transcribed from the cDNA by introducing an RNA polymerase capable of binding to the promoter region.

WO 99/29907 describes methods for making nucleic acids that involve adding a known nucleotide sequence to the 3' end of a first RNA having a known sequence at the 5' end to form a second RNA and reverse transcribing the second RNA to form a cDNA.

### SUMMARY

The present invention provides methods of amplifying viral RNA of unknown sequence from a sample, comprising: (a) adding a poly-A polymerase to a sample of non-polyadenylated viral RNA to attach a poly(A) tract onto the 3' end of the viral RNA; (b) hybridizing an oligonucleotide to the poly(A) tract, wherein said oligonucleotide comprises a hybridization domain comprising a poly(T) tract and a functional domain comprising an RNA polymerase promoter region; (c) synthesizing double stranded cDNA from the viral RNA and oligonucleotide, wherein synthesizing double stranded cDNA from the viral RNA and oligonucleotide comprises reverse transcribing a first strand of said cDNA and synthesizing a second strand of said DNA; (d) amplifying the double stranded cDNA with an RNA polymerase to yield amplified antisense RNA; and (e) converting the amplified antisense RNA into amplified cDNA. In some embodiments, said hybridization domain is at the 3' end of said oligonucleotide and said functional domain is at the 5' end of said oligonucleotide. In some embodiments, said RNA polymerase promoter region comprises a T7 promoter region. In some embodiments, said second strand of said DNA is synthesized using RNase H and DNA polymerase. In some embodiments, the amplified antisense RNA is at least a 50-fold amplification of said double stranded cDNA. In some embodiments, said amplified antisense RNA is at least a 100-fold amplification of said double stranded cDNA. In some embodiments, said amplified antisense RNA is at least a 500-fold amplification of said double stranded cDNA. In some embodiments, the amplified antisense RNA is at least a 1000-fold amplification of said double stranded cDNA. In some embodiments, converting the amplified antisense RNA into amplified cDNA comprises reverse transcription. In some embodiments, said sample comprises a biological, environmental, and/or forensic sample.

The present invention also provides methods of sequencing a viral RNA of unknown sequence from a sample, comprising: (1) amplifying said viral RNA by: (a) adding a poly-A polymerase to a sample of non-polyadenylated viral RNA to attach a poly(A) tract onto the 3' end of the viral RNA, (b) hybridizing an oligonucleotide to the poly(A) tract, wherein said oligonucleotide comprises a hybridization domain comprising a poly(T) tract and a functional domain comprising an RNA polymerase promoter region, (c) synthesizing double stranded cDNA from the viral RNA and oligonucleotide, wherein synthesizing double stranded cDNA from the viral RNA and oligonucleotide comprises reverse transcribing a first strand of said cDNA and synthesizing a second strand of said DNA, (d) amplifying the double stranded cDNA with an RNA polymerase to yield amplified antisense RNA, and (e) converting the amplified antisense RNA into amplified cDNA; and (2) sequencing said viral RNA using the amplified cDNA. In some embodiments, sequencing is by a Next Generation Sequencing technique.

Also disclosed herein are kits for performing the sequencing (e.g., next generation sequencing) and/or RNA amplification (e.g., polyadenylation, reverse transcription, RNA synthesis, and reverse transcription) methods of the invention.

### DETAILED DESCRIPTION

The present invention relates to methods for tagging, amplifying, purifying, and/or characterizing of ribonucleic acid (RNA) in a sample. The present invention relates to methods for tagging a target RNA with a nucleic acid tag, and using that tag to amplify the target RNA for downstream analysis. The present invention relates to the use of poly-A polymerase to attach a poly(A) tail to RNA (e.g., viral genomic material, etc.) within a sample (e.g., biological, clinical, forensic, environmental, research, etc.). In the invention, RNA in a sample is poly(A) tagged. In the invention, tagged (poly(A) tagged) RNA is amplified (e.g., by T7 RNA polymerase, using the Eberwine procedure, etc.). In some embodiments, the methods of the invention generate a large quantity of nucleic acid from trace levels of RNA
(e.g., viral genomic material) present in a sample (e.g., biological, clinical, forensic, environmental, research, etc.). The present invention involves conversion of RNA (amplified from a sample) into DNA (cDNA). In some embodiments, the present invention provides methods for sequencing (e.g., Next Generation Sequencing) of nucleic acids (e.g., DNA, cDNA). In some embodiments, the present invention provides for the identification and/or characterization of pathogenic RNA viruses in a sample (e.g., by poly(A) tagging, RNA amplification, conversion of RNA to cDNA, and DNA sequencing). In some embodiments, the methods and kits described herein are used for amplifying RNA from a sample (e.g., a sample containing trace amounts of RNA from a sample).

In some embodiments, methods of the present invention comprise one or more of the steps of: (1) polyadenylation of target RNA in a sample by addition ofpoly(A) polymerase (or a functional equivalent) to a sample to produce a poly(A) tail on non-polyadenylated RNA (e.g., viral RNA) within the sample; (2) addition of a reverse transcription primer (RTprimer), comprising a 3'-poly(T) segment and a 5'-prometer region (e.g., RNA polymerase promoter (e.g., T7 RNA polymerase promoter), to the RNA in a sample; (3) hybridization of the 3'-poly(T) segment of a RTprimer to the poly(A) segment of a target RNA; (4) reverse transcription of target RNA from the RTprimer; (5) second strand synthesis (e.g., using RNase H and DNA polymerase I), to yield double-stranded cDNA from the target RNA; (6) blunt-ending of cDNA (e.g., with T4 DNA polymerase); (7) transcription of cDNA by a RNA polymerase (e.g., T7 RNA polymerase) into amplified antisense RNA (aRNA); (8) conversion (via reverse transcription of amplified aRNA into cDNA; and (9) analysis or further manipulation of cDNA (e.g., sequencing). In some embodiments, methods comprise each of the above steps in order. In some embodiments, one or more of the above steps are performed. In some embodiments, the order of the above steps is modified. In some embodiments, steps are carried out using different techniques, components, and or reagents than described above. In some embodiments, modifications, additional steps, reordering of steps, etc. is within the scope of embodiments of the present invention.

In some embodiments, a sample containing RNA is used in the methods herein. In some embodiments, a sample may be any sample that comprises a target RNA molecule. The RNA sample may be obtained from a cell, cell culture, a body fluid, a tissue, an organ, the environment (e.g., oil samples, water samples, and air samples), etc. In certain embodiments, samples comprises a mixture of one or more of eukaryotic RNA, bacterial RNA, and/or viral RNA. In some embodiments, the RNA sample comprises one or more types of viral RNA.

In some embodiments of the disclosure, any suitable nucleic acid (e.g., DNA, RNA, etc.) provides a starting material and/or initial template for the methods herein. In some embodiments of the disclosure, the starting material and/or initial template comprises RNA (e.g., viral RNA, viral genomic RNA, bacterial RNA, human RNA, etc.). In the invention, the starting material and/or initial template comprises viral RNA and/or viral genomic RNA. In some embodiments, viral RNA may be single or double stranded. RNA from any suitable virus (known or unknown) finds use as a target in embodiments of the present invention. In some embodiments, target RNA comprises RNA from a dsRNA virus, positive strand RNA virus, positive-sense ssRNA virus, negative-sense ssRNA virus, or any viruses from families therein. In some embodiments of the disclosure, the starting material and/or initial template comprises bacterial RNA and/or bacterial transcriptome RNA. An initial starting material may be converted from a first type of nucleic acid (e.g., DNA or RNA) to a second type of nucleic acid (e.g., RNA or DNA). In the invention the initial template nucleic acid comprises non-polyadenylated viral RNA.

In some embodiments, a suitable nucleic acid template for a poly(A) polymerase is used (e.g., RNA, viral genomic RNA, etc.). In some embodiments, a sample is used which comprises a suitable template for a poly(A) polymerase (e.g., RNA, viral genomic RNA, etc.). In some embodiments, a sample is used (e.g., clinical, biological, environmental, forensic, etc.) that may contain, or is suspected of containing, one or more templates for a poly(A) polymerase (e.g., viral genomic RNA). In some embodiments, a sample contains RNA from one or more viral genomes (e.g., complete genomes, partial genomes, etc.). In some embodiments, viral genomic RNA lacks a poly(A) tail.

In some embodiments, a nucleic acid tag is added to a target RNA. In some embodiments, a nucleic acid tag is added to the 3' end of a target RNA. In some embodiments, a nucleic acid tag is chemically or enzymatically added. In some embodiments, a nucleic acid tag comprises a hybridization site for one or more oligonucleotides (e.g., for subsequent steps herein). In embodiments described herein, the nucleic acid tag comprises, consists of, or consists essentially of a poly(A) tract (e.g., 5-500 A nucleotides, 20-250 A nucleotides, etc.). It should be noted that embodiments that are described herein in conjunction with a poly(A) tag can also be implemented with nucleic acid tags of other sequences. The scope of the disclosure herein should not be limited by the type of tag (e.g., poly(A)) described in conjunction with any one embodiment described herein.

In some embodiments, primers and other oligonucleotides suitable for carrying out steps of the present methods are used. In some embodiments, oligonucleotides hybridize to a portion (e.g., poly(A) tail) of target nucleic acids. In some embodiments, at least a portion (e.g., 3' end) of an oligonucleotide hybridizes to at least a portion (e.g., poly(A) tail) of a target nucleic acid. In some embodiments, at least a portion (e.g., 5' end) of an oligonucleotide is non-complementary to the target. In some embodiments, oligonucleotides are used comprising a poly(T) segment. In some embodiments, the 3' portion of an oligonucleotide comprises a poly(T) segment. In some embodiments, oligonucleotides are used comprising an RNA polymerase promoter region. In some embodiments, the 5' portion of an oligonucleotide comprises an RNA polymerase promoter region. In some embodiments, oligonucleotides are used comprising a poly(T) segment at the 3'-most end and a RNA polymerase promoter region at or near the 5' end.

In some embodiments, oligonucleotides and/or nucleic acids produced by steps described herein comprise at least one amplification domain. As used herein, an amplification domain will primarily be a sequence that can support the amplification of a nucleic acid that comprises such sequence (e.g., by PCR, by RNA polymerase, etc.). Use of nucleic acid sequences in amplification reactions is well known in the art, and non-limiting examples are described herein. In some embodiments, an amplification domain will comprise a sequence that can support primer binding and extension. Standard rules for primer design apply (Sambrook, 1994). In some embodiments, an amplification domain will preferably comprise a primer binding sites for PCR amplification. Parameters for primer design for PCR are well known in the art (see, e.g., Beasley et al, 1999). Primer binding sites for other types of amplification methods are also contemplated. In some embodiments, an amplification domain comprises a promoter sequence (e.g., for an RNA polymerase (e.g., T7 RNA polymerase)). In some embodiments, a promoter sequence (a "transcription domain") is a nucleic acid sequence to which an RNA polymerase binds to initiate transcription. In some embodiments, an amplification domain on a single stranded nucleic acid comprises a single strand of the promoter region (e.g., for an RNA polymerase (e.g., T7 RNA polymerase)). In some embodiments, an oligonucleotide is used that comprises the second strand of a promoter region (e.g., for an RNA polymerase (e.g., T7 RNA polymerase)). In some embodiments, an oligonucleotide also comprises other functional domains (e.g., tagging domains, hybridization domains (e.g., poly(T), etc.). In some embodiments, an oligonucleotide comprising a strand of a promoter region is hybridized to a nucleic acid comprising the second strand of a promoter region to produce a double stranded promoter region (e.g., for an RNA polymerase (e.g., T7 RNA polymerase)).

In certain embodiments of the invention, the methods comprise depleting DNA from a sample (e.g., prior to poly(A) tagging of target RNA, prior to RT of target, prior to amplification of target RNA, etc.). Methods of depleting DNA or separating DNA from RNA are well known to those skilled in the art. One common approach for depleting DNA is to incubate the sample with DNase. Another method for separating DNA from RNA is lithium chloride precipitation. Filter based RNA isolation systems are also known in the art.

In some embodiments of the invention, the methods comprise depleting polyadenylated mRNA from a sample prior to poly(A) tagging target RNA. Methods for specifically isolating polyadenylated mRNA from a sample are well known to those of ordinary skill in the art. For example, a common method for isolating polyadenylated mRNA comprises hybridizing the polyadenylated mRNA to a poly(T) oligonucleotide. Typically, the poly(T) oligonucleotide is attached to a surface, such as a column or a bead. After the polyadenylated mRNA is hybridized to the poly(T) oligonucleotide, it can be separated from the sample. For example, if the polyadenylated mRNA is hybridized to the poly(T) oligonucleotide immobilized on a magnetic bead. The beads may then be separated from the sample using a magnet.

In some embodiments, the methods comprise depleting rRNA from the sample. Depending on the composition of the sample, it may be desirable to deplete eukaryotic rRNA, bacterial rRNA, or both. rRNA may hybridized with one or more oligonucleotides complementary to at least a portion of one or more of the 17S rRNA, 18S rRNA, or 28S rRNA or eukaryotes or at least a portion of one or more of the 16S rRNA or 23S rRNA or prokaryotes. The hybridization complexes are then removed from the sample with an appropriate capture system.

Polyadenylation is the addition of a poly(A) tail to an RNA molecule. Polyadenylation is carried out by a poly-A polymerase. Poly-A polymerase is a commercially available enzyme that catalyzes the addition of adenosine to the 3' end of RNA in a sequence independent fashion. In some embodiments, any enzyme suitable for adding a poly(A) tail to the 3' end of an RNA, in a sequence independent manner finds use in the present invention. Poly-A polymerase is also known as: polynucleotide adenylyltransferase, NTP polymerase, RNA adenylating enzyme, AMP polynucleotidylexotransferase, ATP-polynucleotide adenylyltransferase, ATP-polynucleotidylexotransferase, poly(A) synthetase, polyadenylate nucleotidyltransferase, polyadenylate polymerase, polyadenylate synthetase, polyadenylic acid polymerase, polyadenylic polymerase, terminal riboadenylate transferase, poly(A) hydrolase RNA formation factors, PF1, or adenosine triphosphate:ribonucleic acid adenylyltransferase. In some embodiments, any poly(A) polymerase suitable for the addition of adenosines (e.g., >5, >10, >20, >50, etc.) to the 3' end of a initial RNA template (e.g., viral genomic RNA) finds use with embodiments of the present invention. In some embodiments, any poly(A) polymerase suitable for producing a binding site for an RNA polymerase (e.g., T7 RNA polymerase) promoter finds use with embodiments of the present invention. In some embodiments, any polymerase suitable for template independent RNA synthesis finds use with embodiments described herein. In some embodiments, any polymerase suitable for template independent poly(A) synthesis finds use with embodiments described herein. For example, poly(A) polymerases such as poly(A) polymerase I of E. coli or yeast poly(A) polymerase may be used. Poly(A) polymerases from mammalian or viral sources may also be used. Recombinant poly(A) polymerase enzymes may be used in the methods and kits of the present disclosure. Poly(A) polymerase enzymes are described in, for example, Yehudai-Resheff, S. et al. (2000); Mohanty and Kushner (1999); Mohanty and Kushner (2000); Cao and Sarkar (1997); Raynal et al. (1996).

In some embodiments, the methods herein involve amplification of RNA using the Eberwine procedure (Van Gelder et al. Proc Natl Acad Sci USA. 1990 Mar;87(5):1663-7; Eberwine et al. Proc Natl Acad Sci USA. 1992; 89:3010-3014), modified Eberwine procedures, and portions thereof. The Eberwine procedure takes advantage of the fact that eukaryotic mRNA has a naturally occurring poly(A) tail. This procedure uses the poly(A) tail to attach a T7 RNA polymerase promoter to the mRNA followed by extensive RNA amplification with T7 RNA polymerase.

In some embodiments, tagging (e.g., 3'-end tagging) of target RNA with a nucleic acid tag (e.g., poly(A) tract) is described (e.g., chemically, enzymatically). In some embodiments, 3' polyadenylation is performed by a poly(A)) polymerase (or a functional equivalent) to a sample to produce a poly(A) tail on non-polyadenylated RNA (e.g., viral RNA). Methods for adding nucleic acid sequences to RNA molecules are known to those of skill in the art. For example, in some embodiments a poly(A) polymerase, such as poly(A) polymerase I of E. coli or yeast poly(A) polymerase, is used to add a poly(A) sequence to the 3' end of an RNA.

In some embodiments, complementary DNA (cDNA) is synthesized from an RNA template (e.g., target RNA, polyadenylated target RNA, tagged target RNA, etc.) in a reaction catalyzed by a reverse transcriptase enzyme and a DNA polymerase enzyme. In some embodiments, a reverse transcription primer (RTprimer), reverse transcriptase, and deoxynucleotide triphosphates (A, T, G, C) are added to the sample. In some embodiments, a RT primer comprises a 3' hybridization segment (e.g., for hybridizing to a tagged target RNA) and a 5'-prometer region (e.g., RNA polymerase promoter (e.g., T7 RNA polymerase promoter). In some embodiments, a RT primer comprises a 3'-poly(T) segment and a 5'-promoter region (e.g., RNA polymerase promoter (e.g., T7 RNA polymerase promoter). In some embodiments, a DNA strand is synthesized that is complementary to the target RNA. In some embodiments, a hybrid DNA/RNA (one stand of each) is produced. In some embodiments, to synthesize an additional DNA strand (to produce a duplex cDNA), the target RNA of the hybrid strand is digested (e.g., using an enzyme like RNase H). After digestion of the RNA, a single stranded DNA (ssDNA) remains. In some embodiments, because single stranded nucleic acids are hydrophobic, it tends to loop around itself and form a hairpin loop at the 3' end. In some embodiments, from the hairpin loop, a DNA polymerase can then use it as a primer to transcribe a complementary sequence from the ss cDNA, resulting in a double stranded cDNA with identical sequence as the target RNA. Any other suitable methods for synthesis of cDNA and/or second strands synthesis are within the scope of embodiments of the present invention (See, e.g., Nature Methods 2, 151-152 (2005); D'Alessio & Gerard. Nucleic Acids Res. 1988 March 25; 16(5 Pt B): 1999-2014). In some embodiments described herein, following synthesis of a first strand of cDNA from an RNA template, second stand synthesis is carried out by any suitable method (e.g., using RNase H and DNA polymerase I) to yield a double stranded cDNA.

Any reverse transcriptase suited to carrying out the methods described herein finds use with the present invention. In some embodiments, the reverse transcriptase is Moloney murine leukemia virus (MMLV) reverse transcriptase or avian myeloblastosis virus (AMV) reverse transcriptase. The reverse transcriptase may be a mutant reverse transcriptase, as long as the mutants retain cDNA synthesizing activity. Examples of reverse transcriptase mutants include those with reduced or absent RnaseH activity (e.g., Superscript.TM. II, Superscript.TM. III, and ThermoScript.TM. (Invitrogen)) and those with enhanced activity at higher temperatures (Superscript III and ThermoScript (Invitrogen)).

In some embodiments, steps are performed to remove overhangs and/or sticky ends from nucleic acids (e.g., cDNA) via a blunt-ending procedure. In some embodiments, steps are performed to remove overhangs and/or sticky ends from cDNA produced by methods described herein via techniques understood in the art. In some embodiments blunt ends are produced on cDNA. Blunt ending may be performed by a variety of enzymes and/or enzyme combinations. For example, T4 DNA ligase will blunt-end DNA by filling in 5' overhangs using its 5'-3' polymerase activity, or clipping off 3' overhangs with its 3'-5' exonuclease activity. Similarly, mung bean nuclease will clip off either 5' or 3' overhangs as an exonuclease. Any other suitable blunt-ending techniques known in the art find use herein.

In some embodiments described herein, cDNAs produced by methods described herein are transcribed by a RNA polymerase (e.g., T7 RNA polymerase) into RNA (e.g., amplified antisense RNA (aRNA)). In some embodiments described herein, multiple copies of aRNA can be produced from a single cDNA via RNA transcription, thereby amplifying the sequence. In some embodiments, a nucleic acid sequence is amplified by processive synthesis of multiple RNA molecules from a single cDNA template, which results in amplified, antisense RNA (aRNA). Methods for the synthesis of aRNA are described in U.S. Pat. Nos. 5,545,522, 5,716,785, and 5,891,636. In some embodiments described herein, these methods involve the incorporation of an RNA polymerase promoter into a cDNA molecule by priming cDNA synthesis with a primer complex comprising a synthetic oligonucleotide containing the promoter. Following synthesis of double-stranded cDNA, a reverse transcriptase is added, and antisense RNA is transcribed from the cDNA template. The amplification, which will typically be at least about 500-fold, but may be at least about 1,000-, 5,000-, 10,000-, 15,000-, or 20,000-fold or more, can be achieved from nanogram quantities or less of cDNA. One advantage that the processive synthesis of aRNA has over PCR is that only one region of shared sequence need be known to synthesize aRNA; PCR generally requires that shared sequences be known both 5'- and 3'- to the region of interest, and that these flanking regions be sufficiently close to allow efficient amplification.

In some embodiments, cDNA is produced that contains a promoter for the SP6, T3, or T7 RNA polymerase. The RNA polymerase used for the transcription must be capable of operably binding to the particular promoter region employed in the promoter-primer complex. A preferred RNA polymerase is that found in bacteriophages, in particular SP6, T3 and T7 phages.

In some embodiments, amplified aRNA created by methods described herein is reverse transcribed into cDNA. Suitable reverse transcriptases are described herein, and suitable conditions for such reverse transcription are within the expertise of those in the field.

A number of template dependent processes are available to amplify sequences present in a given sample. A non-limiting example is the polymerase chain reaction (referred to as PCR) which is described in detail in U.S. Patent Nos. 4,683,195, 4,683,202 and 4,800,159, and in Innis et al, 1988. Other non-limiting methods for amplification of target nucleic acid sequences that may be used in the practice of the present invention are disclosed in U.S. Patent Nos. 5,843,650, 5,846,709, 5,846,783, 5,849,546, 5,849,497, 5,849,547, 5,858,652, 5,866,366, 5,916,776, 5,922,574, 5,928,905, 5,928,906, 5,932,451, 5,935,825, 5,939,291 and 5,942,391, GB Application No. 2 202 328, and in PCT Application No. PCT/US89/01025.

In some embodiments, a reverse transcriptase PCR amplification procedure may be performed to amplify RNA populations. Methods of reverse transcribing RNA into cDNA are known (see Sambrook, 1989). Alternative methods for reverse transcription utilize thermostable DNA polymerases. These methods are described in WO 90/07641. Additionally, representative methods of RT-PCR are described in U.S. Patent No. 5,882,864.

Other non-limiting nucleic acid amplification procedures include transcription-based amplification systems (TAS), including nucleic acid sequence based amplification (NASBA) and 3SR (Kwoh et al, 1989; Gingeras et al, PCT Application WO 88/10315). European Application No. 329 822 discloses a nucleic acid amplification process involving cyclically synthesizing single-stranded RNA ("ssRNA"), ssDNA, and double-stranded DNA (dsDNA), which may be used in accordance with the present invention.

Nucleic Acid Sequence Based Amplification (NASBA) (Guatelli, 1990; Compton, 1991) makes use of three enzymes, avian myeloblastosis vims reverse transcriptase (AMV-RT), E. coli RNase H, and T7 RNA polymerase to induce repeated cycles of reverse transcription and RNA transcription. The NASBA reaction begins with the priming of first strand cDNA synthesis with a gene specific oligonucleotide (primer 1) comprising a T7 RNA polymerase promoter. RNase H digests the RNA in the resulting DNA:RNA duplex providing access of an upstream target specific primer(s) (primer 2) to the cDNA copy of the specific RNA target(s). AMV-RT extends the second primer, yielding a double stranded cDNA segment (ds DNA) with a T7 polymerase promoter at one end. This cDNA serves as a template for T7 RNA polymerase that will synthesize many copies of RNA in the first phase of the cyclical NASBA reaction. The RNA then serve as templates for a second round of reverse transcription with the second gene specific primer, ultimately producing more DNA templates that support additional transcription. In some embodiments, NASBA may be adapted to the present invention to provide amplification of target sequences.

In certain embodiments, Strand Displacement Amplification (SDA) is used. SDA is an isothermal amplification scheme that includes five steps: binding of amplification primers to a target sequence, extension of the primers by an exonuclease deficient polymerase incorporating an alpha-thio deoxynucleoside triphosphate, nicking of the hemiphosphorothioate double stranded nucleic acid at a restriction site, dissociation of the restriction enzyme from the nick site, and extension from the 3' end of the nick by an exonuclease deficient polymerase with displacement of the downstream non-template strand. Nicking, polymerization and displacement occur concurrently and continuously at a constant temperature because extension from the nick regenerates another hemiphosphorothioate restriction site. In embodiments wherein primers to both strands of a double stranded target sequence are used, amplification is exponential, as the sense and antisense strands serve as templates for the opposite primer in subsequent rounds of amplification. In some embodiments, SDA may be adapted to the present invention to provide amplification of target sequences.

In certain embodiments, amplification by RNA transcription is used. DNA molecules with promoters can be templates for any one of a number of RNA polymerases (Sambrook 1989). An efficient in vitro transcription reaction can convert a single DNA template into hundreds and even thousands of RNA transcripts. While this level of amplification is orders of magnitude less than what is achieved by some other amplification methods, it is sufficient for amplification of nucleic acids, and provides nucleic acid synthesis without the requirement for opposing primers at either end of a desired sequence.

In some embodiments, methods and kits disclosed herein find use with additional molecular biological techniques for or involving nucleic acid analysis. In some embodiments, nucleic acid sequences (e.g., single nucleotide polymorphisms, mutations, full sequence, etc.) are detected and/or determined by any suitable methods (e.g., nucleic acid detection assay, nucleic acid sequencing, nucleic acid hybridization, etc.). The scope of the present invention is not limited by the application, methods, or techniques with which they find use (e.g., nucleic acid detection, nucleic acid sequencing, nucleic acid identification, etc.).

In some embodiments, compositions and methods described herein find use with nucleic acid sequencing (e.g., production of starting material for sequencing from trace amounts of viral RNA). In some embodiments, methods provided herein are used to prepare a nucleic acid template from a sample for use in nucleic acid sequencing. In some embodiments, nucleic acid is detected and/or the sequence of a nucleic acid is determined by nucleic acid sequencing. In some embodiments, nucleic acid is sequenced using any type of suitable sequencing technology. The present invention is not limited by the type of sequencing method employed. Exemplary sequencing methods are described below. Illustrative non-limiting examples of nucleic acid sequencing techniques include, but are not limited to, chain terminator (Sanger) sequencing, dye terminator sequencing, and next generation sequencing methods.

Chain terminator sequencing uses sequence-specific termination of a DNA synthesis reaction using modified nucleotide substrates. Extension is initiated at a specific site on the template DNA by using a short radioactive, or other labeled, oligonucleotide primer complementary to the template at that region. The oligonucleotide primer is extended using a DNA polymerase, standard four deoxynucleotide bases, and a low concentration of one chain terminating nucleotide, most commonly a di-deoxynucleotide. This reaction is repeated in four separate tubes with each of the bases taking turns as the di-deoxynucleotide. Limited incorporation of the chain terminating nucleotide by the DNA polymerase results in a series of related DNA fragments that are terminated only at positions where that particular di-deoxynucleotide is used. For each reaction tube, the fragments are size-separated by electrophoresis in a slab polyacrylamide gel or a capillary tube filled with a viscous polymer. The sequence is determined by reading which lane produces a visualized mark from the labeled primer as you scan from the top of the gel to the bottom.

Dye terminator sequencing alternatively labels the terminators. Complete sequencing can be performed in a single reaction by labeling each of the di-deoxynucleotide chain-terminators with a separate fluorescent dye, which fluoresces at a different wavelength.

A set of methods referred to as "next-generation sequencing" techniques have emerged as alternatives to Sanger and dye-terminator sequencing methods (Voelkerding et al., Clinical Chem., 55: 641-658, 2009; MacLean et al., Nature Rev. Microbiol., 7: 287-296). Next-generation sequencing technology allows for de novo sequencing of whole genomes to determine the primary nucleic acid sequence of an organism. Next-generation sequencing technology also provide targeted re-sequencing (deep sequencing) which allows for sensitive mutation detection within a population of wild-type sequence. Some examples include recent work describing the identification of HIV drug-resistant variants as well as EGFR mutations for determining response to anti-TK therapeutic drugs. Publications describing the next-generation sequencing permit the simultaneous sequencing of multiple samples during a typical sequencing run including, for example: Margulies, M. et al. "Genome Sequencing in Microfabricated High-Density Picolitre Reactors", Nature, 437, 376-80 (2005); Mikkelsen, T. et al. "Genome-Wide Maps of Chromatin State in Pluripotent and Lineage-Committed Cells", Nature, 448, 553-60 (2007); McLaughlin, S. et al. "Whole-Genome Resequencing with Short Reads: Accurate Mutation Discovery with Mate Pairs and Quality Values", ASHG Annual Meeting (2007); Shendure J. et al. "Accurate Multiplex Polony Sequencing of an Evolved Bacterial Genome", Science, 309, 1728-32 (2005); Harris, T. et al. "Single-Molecule DNA Sequencing of a Viral Genome", Science, 320, 106-9 (2008); Simen, B. et al. "Prevalence of Low Abundance Drug Resistant Variants by Ultra Deep Sequencing in Chronically HIV-infected Antiretroviral (ARV) Naive Patients and the Impact on Virologic Outcomes", 16th International HIV Drug Resistance Workshop, Barbados (2007); Thomas, R. et al. "Sensitive Mutation Detection in Heterogeneous Cancer Specimens by Massively Parallel Picoliter Reactor Sequencing", Nature Med., 12, 852-855 (2006); Mitsuya, Y. et al. "Minority Human Immunodeficiency Virus Type 1 Variants in Antiretroviral-Naive Persons with Reverse Transcriptase Codon 215 Revertant Mutations", J. Vir., 82, 10747-10755 (2008); Binladen, J. et al. "The Use of Coded PCR Primers Enables High-Throughput Sequencing of Multiple Homolog Amplification Products by 454 Parallel Sequencing", PLoS ONE, 2, e197 (2007); and Hoffmann, C. et al. "DNA Bar Coding and Pyrosequencing to Identify Rare HIV Drug Resistance Mutations", Nuc. Acids Res., 35, e91 (2007).

Compared to traditional Sanger sequencing, next-gen sequencing technology produces large amounts of sequencing data points. A typical run can easily generate tens to hundreds of megabases per run, with a potential daily output reaching into the gigabase range. This translates to several orders of magnitude greater than a standard 96-well plate, which can generate several hundred data points in a typical multiplex run. Target amplicons that differ by as little as one nucleotide can easily be distinguished, even when multiple targets from related species or organisms are present. This greatly enhances the ability to do accurate genotyping. Next-gen sequence alignment software programs used to produce consensus sequences can easily identify novel point mutations, which could result in new strains with associated drug resistance. The use of primer bar coding also allows multiplexing of different patient samples within a single sequencing run.

Next-generation sequencing (NGS) methods share the common feature of massively parallel, high-throughput strategies, with the goal of lower costs in comparison to older sequencing methods. NGS methods can be broadly divided into those that require template amplification and those that do not. Amplification-requiring methods include pyrosequencing commercialized by Roche as the 454 technology platforms (e.g., GS 20 and GS FLX), the Solexa platform commercialized by Illumina, and the Supported Oligonucleotide Ligation and Detection (SOLiD) platform commercialized by Applied Biosystems. Non-amplification approaches, also known as single-molecule sequencing, are exemplified by the HeliScope platform commercialized by Helicos BioSciences, and emerging platforms commercialized by VisiGen and Pacific Biosciences, respectively.

In pyrosequencing (Voelkerding et al., Clinical Chem., 55: 641-658, 2009; MacLean et al., Nature Rev. Microbiol., 7: 287-296; U.S. Pat. No. 6,210,891; U.S. Pat. No. 6,258,568), template DNA is fragmented, end-repaired, ligated to adaptors, and clonally amplified in-situ by capturing single template molecules with beads bearing oligonucleotides complementary to the adaptors. Each bead bearing a single template type is compartmentalized into a water-in-oil microvesicle, and the template is clonally amplified using a technique referred to as emulsion PCR. The emulsion is disrupted after amplification and beads are deposited into individual wells of a picotitre plate functioning as a flow cell during the sequencing reactions. Ordered, iterative introduction of each of the four dNTP reagents occurs in the flow cell in the presence of sequencing enzymes and luminescent reporter such as luciferase. In the event that an appropriate dNTP is added to the 3' end of the sequencing primer, the resulting production of ATP causes a burst of luminescence within the well, which is recorded using a CCD camera. It is possible to achieve read lengths greater than or equal to 400 bases, and 1x10⁶ sequence reads can be achieved, resulting in up to 500 million base pairs (Mb) of sequence.

In the Solexa/Illumina platform (Voelkerding et al., Clinical Chem., 55: 641-658, 2009; MacLean et al., Nature Rev. Microbiol., 7: 287-296; U.S. Pat. No. 6,833,246; U.S. Pat. No. 7,115,400; U.S. Pat. No. 6,969,488), sequencing data are produced in the form of shorter-length reads. In this method, single-stranded fragmented DNA is end-repaired to generate 5'-phosphorylated blunt ends, followed by Klenow-mediated addition of a single A base to the 3' end of the fragments. A--addition facilitates addition of T- overhang adaptor oligonucleotides, which are subsequently used to capture the template-adaptor molecules on the surface of a flow cell that is studded with oligonucleotide anchors. The anchor is used as a PCR primer, but because of the length of the template and its proximity to other nearby anchor oligonucleotides, extension by PCR results in the "arching over" of the molecule to hybridize with an adjacent anchor oligonucleotide to form a bridge structure on the surface of the flow cell. These loops of DNA are denatured and cleaved. Forward strands are then sequenced with reversible dye terminators. The sequence of incorporated nucleotides is determined by detection of post-incorporation fluorescence, with each fluor and block removed prior to the next cycle of dNTP addition. Sequence read length ranges from 36 nucleotides to over 50 nucleotides, with overall output exceeding 1 billion nucleotide pairs per analytical run.

Sequencing nucleic acid molecules using SOLID technology (Voelkerding et al., Clinical Chem., 55: 641-658, 2009; MacLean et al., Nature Rev. Microbiol., 7: 287-296; U.S. Pat. No. 5,912,148; U.S. Pat. No. 6,130,073) also involves fragmentation of the template, ligation to oligonucleotide adaptors, attachment to beads, and clonal amplification by emulsion PCR. Following this, beads bearing template are immobilized on a derivatized surface of a glass flow-cell, and a primer complementary to the adaptor oligonucleotide is annealed. However, rather than utilizing this primer for 3' extension, it is instead used to provide a 5' phosphate group for ligation to interrogation probes containing two probe-specific bases followed by 6 degenerate bases and one of four fluorescent labels. In the SOLID system, interrogation probes have 16 possible combinations of the two bases at the 3' end of each probe, and one of four fluors at the 5' end. Fluor color and thus identity of each probe corresponds to specified color-space coding schemes. Multiple rounds (usually 7) of probe annealing, ligation, and fluor detection are followed by denaturation, and then a second round of sequencing using a primer that is offset by one base relative to the initial primer. In this manner, the template sequence can be computationally re-constructed, and template bases are interrogated twice, resulting in increased accuracy. Sequence read length averages 35 nucleotides, and overall output exceeds 4 billion bases per sequencing run.

In certain embodiments, nanopore sequencing in employed (see, e.g., Astier et al., J Am Chem Soc. 2006 Feb. 8; 128(5):1705-10). The theory behind nanopore sequencing has to do with what occurs when the nanopore is immersed in a conducting fluid and a potential (voltage) is applied across it: under these conditions a slight electric current due to conduction of ions through the nanopore can be observed, and the amount of current is exceedingly sensitive to the size of the nanopore. If DNA molecules pass (or part of the DNA molecule passes) through the nanopore, this can create a change in the magnitude of the current through the nanopore, thereby allowing the sequences of the DNA molecule to be determined.

HeliScope by Helicos BioSciences (Voelkerding et al., Clinical Chem., 55: 641-658, 2009; MacLean et al., Nature Rev. Microbiol., 7: 287-296; U.S. Pat. No. 7,169,560; U.S. Pat. No. 7,282,337; U.S. Pat. No. 7,482,120; U.S. Pat. No. 7,501,245; U.S. Pat. No. 6,818,395; U.S. Pat. No. 6,911,345; U.S. Pat. No. 7,501,245) does not require clonal amplification. Template DNA is fragmented and polyadenylated at the 3' end, with the final adenosine bearing a fluorescent label. Denatured polyadenylated template fragments are ligated to poly(dT) oligonucleotides on the surface of a flow cell. Initial physical locations of captured template molecules are recorded by a CCD camera, and then label is cleaved and washed away. Sequencing is achieved by addition of polymerase and serial addition of fluorescently-labeled dNTP reagents. Incorporation events result in fluor signal corresponding to the dNTP, and signal is captured by a CCD camera before each round of dNTP addition. Sequence read length ranges from 25-50 nucleotides, with overall output exceeding 1 billion nucleotide pairs per analytical run.

Other emerging single molecule sequencing methods include real-time sequencing by synthesis using a VisiGen platform (Voelkerding et al., Clinical Chem., 55: 641-658, 2009; U.S. Pat. No. 7,329,492; U.S. patent application Ser. No. 11/671,956; U.S. patent application Ser. No. 11/781,166) in which immobilized, primed DNA template is subjected to strand extension using a fluorescently-modified polymerase and florescent acceptor molecules, resulting in detectible fluorescence resonance energy transfer (FRET) upon nucleotide addition.

Another real-time single molecule sequencing system developed by Pacific Biosciences (Voelkerding et al., Clinical Chem., 55: 641-658, 2009; MacLean et al., Nature Rev. Microbiol., 7: 287-296; U.S. Pat. No. 7,170,050; U.S. Pat. No. 7,302,146; U.S. Pat. No. 7,313,308; U.S. Pat. No. 7,476,503) utilizes reaction wells 50-100 nm in diameter and encompassing a reaction volume of approximately 20 zeptoliters (10x10⁻²¹ L). Sequencing reactions are performed using immobilized template, modified phi29 DNA polymerase, and high local concentrations of fluorescently labeled dNTPs. High local concentrations and continuous reaction conditions allow incorporation events to be captured in real time by fluor signal detection using laser excitation, an optical waveguide, and a CCD camera.

In some embodiments, compositions and methods described herein find use with nucleic acid detection, characterization, and/or identification. In some embodiments, methods provided herein are used to prepare a nucleic acid template from a sample for use in subsequent analysis. In some embodiments, nucleic acid is detected, characterized, and/or identified using any suitable technology. The present invention is not limited by the type of nucleic acid analysis methods employed. Exemplary methods are described below.

In some embodiments, nucleic acid is detected by hybridization to an oligonucleotide probe. A variety of hybridization assays using a variety of technologies for hybridization and detection are available. For example, in some embodiments, TaqMan assay (PE Biosystems, Foster City, Calif.; See e.g., U.S. Pat. Nos. 5,962,233 and 5,538,848) is utilized. The assay is performed during a PCR reaction. The TaqMan assay exploits the 5'-3' exonuclease activity of the AMPLITAQ GOLD DNA polymerase. A probe composed of an oligonucleotide with a 5'-reporter dye (e.g., a fluorescent dye) and a 3'-quencher dye is included in the PCR reaction. During PCR, if the probe is bound to its target, the 5'-3' nucleolytic activity of the AMPLITAQ GOLD polymerase cleaves the probe between the reporter and the quencher dye. The separation of the reporter dye from the quencher dye results in an increase of fluorescence. The signal accumulates with each cycle of PCR and can be monitored with a fluorimeter.

In some embodiments, nucleic acid is detected by Northern blot analysis (e.g., following amplification by the methods described herein). Northern blot analysis involves the separation of nucleic acid and hybridization of a complementary labeled probe.

In some embodiments, nucleic acid is detected using a detection assay (e.g., following amplification by the methods described herein) including, but not limited to, enzyme mismatch cleavage methods (e.g., Variagenics, U.S. Pat. Nos. 6,110,684, 5,958,692, 5,851,770); polymerase chain reaction; branched hybridization methods (e.g., Chiron, U.S. Pat. Nos. 5,849,481, 5,710,264, 5,124,246, and 5,624,802); rolling circle replication (e.g., U.S. Pat. Nos. 6,210,884, 6,183,960 and 6,235,502); NASBA (e.g., U.S. Pat. No. 5,409,818); molecular beacon technology (e.g., U.S. Pat. No. 6,150,097); E-sensor technology (Motorola, U.S. Pat. Nos. 6,248,229, 6,221,583, 6,013,170, and 6,063,573); cycling probe technology (e.g., U.S. Pat. Nos. 5,403,711, 5,011,769, and 5,660,988); Dade Behring signal amplification methods (e.g., U.S. Pat. Nos. 6,121,001, 6,110,677, 5,914,230, 5,882,867, and 5,792,614); ligase chain reaction (Barnay Proc. Natl. Acad. Sci USA 88, 189-93 (1991)); FULL-VELOCITY assays; and sandwich hybridization methods (e.g., U.S. Pat. No. 5,288,609). In other embodiments, the detection assay employed is the INVADER assay (Third Wave Technologies) which is described in U.S. Pat. Nos. 5,846,717, 5,985,557, 5,994,069, 6,001,567, and 6,090,543, WO 97/27214, WO 98/42873, Lyamichev et al., Nat. Biotech., 17:292 (1999), and Hall et al., PNAS, USA, 97:8272 (2000).

Any of the compositions described herein may be provided in a kit. In a non-limiting example the kit, in suitable container means, comprises, one or more: a poly(A) polymerase, reverse transcriptase, RNA polymerase, nucleotides; buffer; control RNA, oligonucleotide, etc. Any of the other reagents discussed herein or implied by the methods described may also be included in a kit.

## Claims

1. A method of amplifying viral RNA of unknown sequence from a sample, comprising:
(a) adding a poly-A polymerase to a sample of non-polyadenylated viral RNA to attach a poly(A) tract onto the 3' end of the viral RNA;
(b) hybridizing an oligonucleotide to the poly(A) tract, wherein said oligonucleotide comprises a hybridization domain comprising a poly(T) tract and a functional domain comprising an RNA polymerase promoter region;
(c) synthesizing double stranded cDNA from the viral RNA and oligonucleotide, wherein synthesizing double stranded cDNA from the viral RNA and oligonucleotide comprises reverse transcribing a first strand of said cDNA and synthesizing a second strand of said DNA;
(d) amplifying the double stranded cDNA with an RNA polymerase to yield amplified antisense RNA; and
(e) converting the amplified antisense RNA into amplified cDNA.

2. The method of claim 1, wherein said hybridization domain is at the 3' end of said oligonucleotide and said functional domain is at the 5' end of said oligonucleotide.

3. The method of claim 1, wherein said RNA polymerase promoter region comprises a T7 promoter region.

4. The method of claim 1, wherein said second strand of said DNA is synthesized using RNase H and DNA polymerase.

5. The method of claim 1, wherein said amplified antisense RNA:
(i) is at least a 50-fold amplification of said double stranded cDNA;
(ii) is at least a 100-fold amplification of said double stranded cDNA;
(iii) is at least a 500-fold amplification of said double stranded cDNA; and/or
(iv) is at least a 1000-fold amplification of said double stranded cDNA.

6. The method of claim 1, wherein converting the amplified antisense RNA into amplified cDNA comprises reverse transcription.

7. The method of claim 1, wherein said sample comprises a biological, environmental, and/or forensic sample.

8. The method of claim 1, further comprising blunt-ending of cDNA.

9. The method of claim 1, further comprising depleting DNA from the sample.

10. The method of claim 1, further comprising depleting polyadenylated mRNA from the sample prior to poly(A) tagging target RNA.

11. The method of claim 1, wherein the poly-A polymerase is poly(A) polymerase I of *E. coli* or yeast poly(A) polymerase.

12. A method of sequencing a viral RNA of unknown sequence from a sample, comprising:
(1) amplifying said viral RNA by the method of claim 1; and
(2) sequencing said viral RNA using the amplified cDNA.

13. The method of claim 12, wherein said sequencing is by a Next Generation Sequencing technique.

## Patentansprüche

1. Verfahren zum Amplifizieren von Virus-RNA mit unbekannter Sequenz aus einer Probe, umfassend:
(a) Zugeben einer poly-A-Polymerase zu einer Probe von nichtpolyadenylierter Virus-RNA, um einen poly(A)-Abschnitt an das 3'-Ende der Virus-RNA anzuheften;
(b) Hybridisieren eines Oligonukleotids an den poly(A)-Abschnitt, wobei das Oligonukleotid eine Hybridisierungsdomäne umfassend einen poly(T)-Abschnitt und eine funktionelle Domäne umfassend eine RNA-Polymerasepromoterregion umfasst;
(c) Synthetisieren von Doppelstrang-cDNA von der Virus-RNA und dem Oligonukleotid, wobei das Synthetisieren von Doppelstrang-cDNA von der Virus-RNA und dem Oligonukleotid das reverse Transkribieren eines Erststrangs der cDNA und das Synthetisieren eines Zweitstrangs der DNA umfasst;
(d) Amplifizieren der Doppelstrang-cDNA mit einer RNA-Polymerase, um zu amplifizierter Antisense-RNA zu gelangen; und
(e) Umwandeln der amplifizierten Antisense-RNA in amplifizierte cDNA.

2. Verfahren nach Anspruch 1, wobei sich die Hybridisierungsdomäne am 3'-Ende des Oligonukleotids befindet und sich die funktionelle Domäne am 5'-Ende des Oligonukleotids befindet.

3. Verfahren nach Anspruch 1, wobei die RNA-Polymerasepromoterregion eine T7-Promoterregion umfasst.

4. Verfahren nach Anspruch 1, wobei der Zweitstrang der DNA unter Verwendung von RNase H und DNA-Polymerase synthetisiert wird.

5. Verfahren nach Anspruch 1, wobei die amplifizierte Antisense-RNA:
(i) mindestens eine 50-fache Amplifikation der Doppelstrang-cDNA ist;
(ii) mindestens eine 100-fache Amplifikation der Doppelstrang-cDNA ist;
(iii) mindestens eine 500-fache Amplifikation der Doppelstrang-cDNA ist; und/oder
(iv) mindestens eine 1000-fache Amplifikation der Doppelstrang-cDNA ist.

6. Verfahren nach Anspruch 1, wobei das Umwandeln der amplifizierten Antisense-RNA in amplifizierte cDNA reverse Transkription umfasst.

7. Verfahren nach Anspruch 1, wobei die Probe eine biologische Probe, eine Umweltprobe und/oder eine gerichtsmedizinische Probe umfasst.

8. Verfahren nach Anspruch 1, das weiterhin das Abstumpfen der cDNA umfasst.

9. Verfahren nach Anspruch 1, das weiterhin das Abreichern von DNA aus der Probe umfasst.

10. Verfahren nach Anspruch 1, das weiterhin das Abreichern von polyadenylierter mRNA aus der Probe umfasst, bevor die Ziel-RNA mit einem poly(A)-Tag versehen wird.

11. Verfahren nach Anspruch 1, wobei es sich bei der poly-A-Polymerase um poly(A)-Polymerase I von *E. coli* oder um Hefe-poly(A)-Polymerase handelt.

12. Verfahren zum Sequenzieren einer Virus-RNA mit unbekannter Sequenz aus einer Probe, umfassend:
(1) Amplifizieren der Virus-RNA nach dem Verfahren nach Anspruch 1; und
(2) Sequenzieren der Virus-RNA unter Verwendung der amplifizierten cDNA.

13. Verfahren nach Anspruch 12, wobei die Sequenzierung mittels einer "Next Generation Sequencing"-Technik erfolgt.

## Revendications

1. Procédé d'amplification d'un ARN viral de séquence inconnue à partir d'un échantillon, comprenant les étapes consistant à :
(a) ajouter une poly-A polymérase à un échantillon d'ARN viral non poly-adénylé pour fixer un tractus de poly(A) sur l'extrémité 3' de l'ARN viral ;
(b) hybrider un oligonucléotide avec le tractus de poly(A), dans lequel ledit oligonucléotide comprend un domaine d'hybridation, qui comprend un tractus de poly(T) ainsi qu'un domaine fonctionnel comprenant une région promoteur d'ARN polymérase ;
(c) synthétiser de l'ADNc en double brin à partir de l'ARN viral et de l'oligonucléotide, dans lequel la synthèse d'un ADNc en double brin à partir de l'ARN viral et de l'oligonucléotide comprend les étapes consistant à transcrire, de manière inverse, un premier brin dudit ADNc et synthétiser un deuxième brin dudit ADN ;
(d) amplifier l'ADNc en double brin avec une ARN polymérase pour produire un ARN antisens amplifié ; et
(e) convertir l'ARN antisens amplifié en un ADNc amplifié.

2. Procédé selon la revendication 1, dans lequel ledit domaine d'hybridation est à l'extrémité 3' dudit oligonucléotide et ledit domaine fonctionnel est à l'extrémité 5' dudit oligonucléotide.

3. Procédé selon la revendication 1, dans lequel ladite région promoteur d'une ARN polymérase comprend une région promoteur de T7.

4. Procédé selon la revendication 1, dans lequel ledit deuxième brin dudit ADN est synthétisé en utilisant une RNase H et une ADN polymérase.

5. Procédé selon la revendication 1, dans lequel ledit ARN antisens amplifié :
(i) est une amplification, d'au moins 50 fois, dudit ADNc en double brin ;
(ii) est une amplification, d'au moins 100 fois, dudit ADNc en double brin ;
(iii) est une amplification, d'au moins 500 fois, dudit ADNc en double brin ; et/ou
(iv) est une amplification, d'au moins 1 000 fois, dudit ADNc en double brin.

6. Procédé selon la revendication 1, dans lequel la conversion de l'ARN antisens amplifié en un ADNc amplifié comprend une transcription inverse.

7. Procédé selon la revendication 1, dans lequel ledit échantillon comprend un échantillon biologique, environnemental et/ou médico-légal.

8. Procédé, selon la revendication 1, comprenant en outre l'étape consistant à rendre l'ADNc à extrémités franches.

9. Procédé, selon la revendication 1, comprenant en outre un épuisement de l'ADN provenant de l'échantillon.

10. Procédé, selon la revendication 1, comprenant en outre l'étape consistant à épuiser l'ARNm poly-adénylé provenant de l'échantillon avant d'étiqueter avec des poly(A) l'ARN cible.

11. Procédé selon la revendication 1, dans lequel la poly-A polymérase est une poly(A) polymérase I d'E. *coli* ou une poly(A) polymérase de levure.

12. Procédé de séquençage d'un ARN viral de séquence inconnue à partir d'un échantillon, comprenant les étapes consistant à :
(1) amplifier ledit ARN viral par le procédé selon la revendication 1 ; et
(2) séquencer ledit ARN viral en utilisant l'ADNc amplifié.

13. Procédé selon la revendication 12, dans lequel ledit séquençage se fait par une technique de séquençage de nouvelle génération.
